# EUROPEAN PATENT APPLICATION

(11) **EP 4 641 511 A1**
(43) Date of publication of application: **29.10.2025**
(21) Application number: 24172174.5
(22) Date of filing: 24.04.2024
(51) Int. Cl.: G06T 19/20, G16H 50/00, G06T 19/00

(54) **METHOD FOR PROVIDING AN IMAGE SIGNAL FOR A MEDICAL VISUALIZATION SYSTEM AND MEDICAL VISUALIZATION SYSTEM**

(71) Applicant: Carl Zeiss Meditec AG, 07745 Jena (DE)
(72) Inventor: Philipp, Markus, 73447 Oberkochen (DE); You, Fang, 07745 Jena (DE); Geissler, Enrico, 07745 Jena (DE)
(74) Representative: Patentanwälte Bressel und Partner mbB

(57) **Abstract**

The invention relates to a Method for providing an image signal (17) for a medical visualization system (1), comprising: receiving at least one layer (11) of three-dimensional topography data (10) of a patient region of interest (21), receiving three-dimensional preoperative data (12) of the patient region of interest (21), determining distance values (14) of a distance (13) between the three-dimensional preoperative data (12) and the at least one layer (11) of the three-dimensional topography data (10), generating a rendered image (16) by rendering the three-dimensional preoperative data (12) with a rendering style based at least in part on the determined distance values (14), and generating and providing an image signal (17) which represents the rendered image (16). The invention further relates to a medical visualization system (1).

## Description

The invention relates to a method for providing an image signal for a medical visualization system and a medical visualization system.

Medical visualization systems, such as surgical microscopes, do not only provide high-resolution visualization of a surgical scene, but also allow to differentiate tissue by using different modalities (e.g. fluorescence) and to provide a virtual display of invisible or artificial anatomies (e.g. by neuro navigation) inside the viewing field.

For the display of virtual anatomies, such as an outline of a tumor, the three-dimensional object to be displayed is rendered based on three-dimensional preoperative data and a (video) image signal is displayed optically in the beam path of the medical visualization system, e.g. the beam path of the surgical microscope. A projector projects the (video) image signal onto a glass plate, which is placed in a tilted orientation into the beam path. The rendered object may then be viewed by the surgeon as an overlay over a white-light reproduction of the surgical scene. The disadvantages are that the displayed objects do not have the right perspective with respect to the view of the surgical scene through the beam path and the rendered object seems to hover semi-transparently above the surface of the surgical scene.

These drawbacks may be solved by using a stereo medical visualization system, in particular a digital stereo surgical microscope. These allow to render separate images of the three-dimensional object for the left and the right channel, each with its own perspective, such that the surgeon can perceive the rendered object three-dimensionally in a spatially correct way and perspective.

US 2015 / 0 221 105 A1 describes an imaging system and methods for displaying a fused multidimensional reconstructed image. WO 2021/149056 A1 describes a system and a method for improved electronic assisted medical procedures. WO 2022/192690 A1 describes an automated touchless registration for surgical navigation.

The invention is based on the technical problem of developing an enhanced method for providing an image signal for a medical visualization system and an enhanced medical visualization system.

According to the invention, the technical problem is solved by the subject matter of the independent claims. Advantageous embodiments of the invention emerge from the dependent claims.

One of the key ideas of the invention is to take distance values, in particular of each point (vertex, voxel etc.) individually, into account when rendering three-dimensional preoperative data (e.g. a virtual anatomy) of a patient region of interest. The distance values are determined, in particular, to describe an invidual distance between points of the three-dimensional preoperative data of a patient region of interest and at least one layer of a three-dimensional topography data of the patient region of interest. Based at least in part on the determined distance values a rendering style, in particular of each point, of the three-dimensional preoperative data (e.g. the virtual anatomy) is set. This allows the surgeon or another viewer to distinguish the spatial relationship between a three-dimensional overlay of the three-dimensional preoperative data of the patient region of interest and an occluding surface of the surgical scene of the patient region of interest, which is defined by the three-dimensional topography data. In other words, the surgeon or another viewer may perceive, by the different rendering styles, how deep an object within a three-dimensional overlay is below the surface of the patient region of interest or how far an object within the three-dimensional overlay is above the surface of the patient region of interest. The ability to provide such an information is a key advantage of the invention.

In particular, a method is provided for providing an image signal for a medical visualization system, comprising: receiving at least one layer of three-dimensional topography data of a patient region of interest, receiving three-dimensional preoperative data of the patient region of interest, determining distance values of a distance between the three-dimensional preoperative data and the at least one layer of the three-dimensional topography data, generating a rendered image by rendering the three-dimensional preoperative data with a rendering style based at least in part on the determined distance values, and generating and providing an image signal which represents the rendered image.

Further, in particular, a medical visualization system is provided, comprising a processing device, configured to: receive at least one layer of three-dimensional topography data of a patient region of interest, receive three-dimensional preoperative data of the patient region of interest, determine distance values of a distance between the three-dimensional preoperative data and the at least one layer of the three-dimensional topography data, generate a rendered image by rendering the three-dimensional preoperative data with a rendering style based at least in part on the determined distance values, and generate and provide an image signal which represents the rendered image.

Receiving the at least one layer of three-dimensional topography data of the patient region of interest may comprise capturing the at least one layer of three-dimensional topography data of the patient region of interest, for example, by using a stereoscopic image acquisition device (e.g. a stereoscopic camera) and a three-dimensional depth reconstruction. Alternatively, other means and/or methods may be used to capture the three-dimensional topography data of the patient region of interest. Such means and/or methods may be comprise one of the following: intraoperative Optical Coherence Tomography (OCT), lidar (e.g. using a lidar sensor), structured light (e.g. by projecting dots or lines onto the patient region of interest, in particular within the infrared spectrum, and capturing an image, in which the structured light is used to reconstruct the topography). Receiving the at least one layer of three-dimensional topography data of the patient region of interest may comprise determining the three-dimensional topography data of the patient region of interest, for example, by analyzing stereo images of the patient region of interest and/or other sensor data from which depth information can be derived directly or indirectly. In principle, receiving the at least one layer of three-dimensional topography data of the patient region of interest may comprise determining the at least one layer from the three-dimensional preoperative data. To this end, a surface within the three-dimensional preoperative data may be determined, e.g. a surface of the dura.

A layer of the three-dimensional topography data refers, in particular, to three-dimensional information (e.g. a depth map, a depth map in the medical visualization system's or surgical microscope's perspective, a three-dimensional point cloud in the same coordinate as the three-dimensional preoperative data) of a surface of the patient's body. A layer may correspond to one of the following anatomical features: a skull, a dura, a brain tissue, a tumor surface, a skin surface etc. The layer of the three-dimensional topography data may contain a plurality of (three-dimensional) data points.

In particular, the at least one layer of the three-dimensional topography data may be captured by one or more of the methods mentioned above. Alternatively or additionally, the at least one layer may also be set artificially, e.g. by the surgeon or another user. In other words, the at least one layer may be defined artificially by defining a topography.

In particular, the at least one layer of the three-dimensional topography data represents the nearest visible surface to an image acquisition device (e.g. a camera) of the medical visualization system and/or to the viewer with respect to a real image captured by the image acquisition device (e.g. the camera) from the patient region of interest. However, other layers may correspond to other surfaces.

In particular, the three-dimensional topography data and the three-dimensional preoperative data are each represented in the same coordinate system, e.g. the coordinate system of a surgical navigation system or a data or image acquisition device (e.g. a camera) of the medical visualization system or a robotic stand of the medical visualization system. If the data is not represented in the same coordinate system, the data is transformed into the same coordinate system by using an appropriate transformation. In particular, a (patient) registration common in the art may be performed in order to derive the parameters for such a transformation.

Three-dimensional preoperative data may be captured and/or generated, for example, by computed tomography-(CT)-based or magnetic resonance imaging-(MRT)-based procedures. Other, in particular imaging, methods such as ultrasound-based, X-ray-based, fluorescence-based, SPECT-based or PET-based methods may also be used to capture and/or generate the data. SPECT-based or PET-based methods may also be used for capture and/or generation. In general, it is also possible to use three-dimensional intraoperative data, i.e. data that is acquired and/or generated during surgery. In particular, in this disclosure the term three-dimensional preoperative data incorporates the term three-dimensional intraoperative data.

Since different points (or vertices, voxels etc.) of the three-dimensional preoperative data may have different distances to the at least one layer of the three-dimensional topography data, in particular, a distance is determined with respect to each point of the three-dimensional preoperative data of the patient region of interest. In other words: For each point, a distance value is determined and forms at least in part the basis for the rendering style of that point. Therefore, each point (if it is rendered) is rendered with a rendering style based at least in part on the determined distance for that respective point. For example, if an individual distance value d is determined for each of the points, the renderer/shader renders each point based at least in part on the respective distance value d. Therefore each point (vertex, voxel etc.) is processed individually and differently according to its distance to the at least one layer of the three-dimensional topography data. It is also possible to determine the distances only for a selected subset of the points of the three-dimensional preoperative data. The subset may comprise a plurality of points.

The rendered image may be projected onto a glass plate within a beam path of the medical visualization device. Alternatively, the rendered image may be displayed on a display of the medical visualization device, in particular a display of a (digital) surgical microscope.

It is also possible to use the invention with a plurality of patient regions of interest. The method is then carried out in the same way for every patient region of interest.

The medical visualization system may be a surgical microscope or may comprise a surgical microscope, in particular a surgical stereo microscope. The rendered image is then, in particular, generated and provided for each channel of the stereo microscope, taking into account the perspective of each of the channels. The medical visualization system may be used, for example, for surgery, in particular microsurgery, neurosurgery, spinal microsurgery.

Parts of the medical visualization system, in particular the processing device, may be designed individually or collectively as a combination of hardware and software, for example as program code that is executed on at least one microcontroller or microprocessor. However, it is also possible for parts to be designed individually or collectively as an application-specific integrated circuit (ASIC) and/or field-programmable gate array (FPGA). The processing device may comprise at least one processor and at least one memory.

The rendered image may be generated (i.e. rendered) with relation to a virtual image acquisition device, whereby this can be a mathematical or physical and, in particular, computer-aided evaluable optical model of an image acquisition device, e.g. a camera of the medical visualization system. In particular, a computer-implemented calculation of the pixels of the rendered image as virtual image may be performed. Among other things, this virtual image is dependent on parameters of the (modeled) image acquisition device. In particular, the virtual image may be generated as a function of the intrinsic parameters of the image acquisition device for microscopic imaging, especially with these parameters. If corresponding images of a virtual stereo system (e.g. a stereoscopic camera) are generated, these can also be generated as a function of the extrinsic parameters of the two image acquisition devices (e.g. the cameras for the left and the right channel) for microscopic imaging, in particular with these parameters. In other words, the parameters of the image acquisition device(s) of the medical visualization system that are used for (microscopic) imaging can be taken into account when evaluating the model for generating the virtual images. The virtual image may also be generated as a function of a pose, i.e. a position and/or orientation, of the (modeled) image acquisition device of the medical visualization system, e.g. the surgical microscope. In particular, the pose of the image acquisition device(s) of the medical visualization device used for (microscopic) imaging can be taken into account when evaluating the model for generating the virtual images, whereby registration information can be used. Taking into account registration information, it is possible, for example, to determine which pose of the virtual image acquisition device in a (reference) coordinate system, which can also be referred to as the render coordinate system, corresponds to the real pose of the (modeled) image acquisition device of the medical visualization system and this information can be used for the rendering process. In other words, a pose of the at least one virtual image acquisition device can be the same as the pose of the modeled image acquisition device in the (reference) coordinate system. Thus, it is possible to generate a virtual image that corresponds to one of the modeled image acquisition devices both in terms of the parameters and the acquisition pose. For example, an image of a tumor object in the three-dimensional preoperative data to be superimposed can be generated by rendering and can then be provided as rendered image and used for augmentation.

Rendering is performed, in particular, in real-time, i.e. if the position of the medical visualization system is changed with respect to the surgical scene the rendered image changes accordingly.

The image signal is, in particular, a video image signal, i.e. the rendering is performed continuously, such that a sequence of single rendered images is generated, providing frames for the video image signal. Any changes within the surgical scene and/or a pose of the medical visualization system can thus be taken into account in real-time.

In one embodiment, the method further comprises receiving a real image of the patient region of interest, merging the rendered image with the real image, wherein the generated and provided image signal represents the merged image. This allows to provide a single image signal for a real image of the patient region of interest and the rendered image. In particular, this allows to provide an augmented version of the real image, in which the real image is augmented by the content of the rendered image, i.e. with additional information. It is possible to display this single image signal on a display of the medical visualization system. The surgeon or another person may view the merged image on the display. The real image is, in particular, a white-light or RGB image. The real image may, in particular, be a stereoscopic image or a stereoscopic pair of images. In this case, the merged image is a stereoscopic image or a stereoscopic pair of images, providing one image per channel. The merging of the rendered image and the real image may be performed, for example, by alpha blending. In particular, using alpha blending the real image is used as a background while the rendered image is expressed over this background. By this expression a composite image is created, which resembles the merged image. For this purpose, an additional alpha value may be assigned to each of the pixels of the rendered image in order to define transparency values for each of the pixels of the rendered image.

Receiving of the real image of the patient region of interest may comprise capturing the real image by an image acquisition device (e.g. a camera) of the medical visualization system. In case of a stereoscopic image or a stereoscopic pair of images a stereoscopic image acquisition device (e.g. a stereoscopic camera) of the medical visualization system may be used.

In one embodiment, the distance values are determined with respect to a predetermined viewpoint of the medical visualization system. In particular, the predetermined viewpoint corresponds with a viewpoint of the surgeon or another person and/or an image acquisition device (e.g. a camera) of the medical visualization system. By using this reference, the distance is determined in a natural way as it would be perceived by a human observer or the image acquisition device (e.g. the camera). The distance between the at least one layer of the three-dimensional topography data of the patient region of interest and the three-dimensional preoperative data of the patient region of interest may be determined, in particular calculated, by determining point pairs along straight virtual rays originating from the viewpoint and intersecting with the at least one layer of the three-dimensional topography data and a point of the three-dimensional preoperative data, the two intersection points defining coordinates for each point of the point pairs, and then determining the values for the distance along the ray of each point pair using the coordinates. This may be performed for all coordinates. For determining the distance, in particular, a Euclidian metric may be used.

In one embodiment, the distance values are determined as absolute distance between the three-dimensional preoperative data and the at least one layer of the three-dimensional topography data. This allows to use absolute distance values to influence the rendering style of the rendered image. With respect to a point of the three-dimensional preoperative data, the absolute distance is, in particular, defined by the shortest possible distance between this point and any point of the at least one layer of the three-dimensional topography data.

In one embodiment, the distance values are determined along a direction parallel to a predetermined vector. This allows defining a direction of reference for determining the distance. The distance between the at least one layer of the three-dimensional topography data of the patient region of interest and the three-dimensional preoperative data of the patient region of interest may be determined, in particular calculated, by determining point pairs along straight virtual rays running parallel to the predetermined vector and intersecting with the at least one layer of the three-dimensional topography data and a point of the three-dimensional preoperative data, the two intersection points defining coordinates for each point of the point pairs, and determining the values for the distance along the ray of each point pair using the coordinates. This may be performed for all coordinates. For determining the distance, in particular, a Euclidian metric may be used.

In one embodiment, the predetermined vector runs parallel to an optical axis of the medical visualization system. In particular, the optical axis is an optical axis of an image acquisition system (e.g. a camera) of the medical visualization system.

In one embodiment, the rendering style includes an opacity or a transparency, wherein an opacity value or transparency value is set at least in part based on the determined distance values. This allows to directly indicate the distance between the three-dimensional preoperative data and the at least one layer of the three-dimensional topography data in a way that allows the surgeon or another viewer to better distinguish the spatial relationship between the three-dimensional overlay of the three-dimensional preoperative data of the patient region of interest and an occluding surface of the surgical scene of the patient region of interest. Since the distance is directly indicated by the opacity or transparency this allows the surgeon to securely navigate within the patient region of interest. In particular, the opacity value may scale inversely with the distance value, i.e. a larger distance value leads to a smaller opacity value. In particular, a transparency value may scale directly with the distance value, i.e. a larger distance value leads to a higher transparency value. When generating the rendered image, the opacity values or the transparency values are used when merging pixel information from different distances, for example by determining respective opacity values and using alpha blending. For example, if a smaller distance value corresponds to an opacity value of 60 % (and an respective alpha value) and a larger distance value corresponds to an opacity value of 10 % (and an respective alpha value), the value of the alpha channel for the overlapping pixel information derived from the three-dimensional preoperative data of these two distances is a result of the opacity values of 60 % and 10 %, respectively, and will typically be larger than 60 %. In particular, the resulting rendered image contains an alpha channel, which is the merged result of overlapping pixels a and b. Different alpha blending modes may be used, e.g. taking the maximum value, taking the sum of alpha, taking (1-alpha_a)(1-alpha_b), taking the nearest value etc.

In one embodiment, the rendering style includes a color, wherein a color value is set at least in part based on the determined distance values. This allows to directly indicate the distance between the three-dimensional preoperative data and the at least one layer of the three-dimensional topography data in a way that allows the surgeon or another viewer to better distinguish the spatial relationship between the three-dimensional overlay of the three-dimensional preoperative data of the patient region of interest and an occluding surface of the surgical scene of the patient region of interest. Since the distance is directly indicated by the color this allows the surgeon to securely navigate within the patient region of interest. For example, a range of colors may be defined which corresponds to distance values. The range of colors may correspond to a single color with different brightness values or the range of colors may correspond to different colors which are mapped to the distance values, e.g. within a given range of distance values the color changes from red to blue etc.

In one embodiment rendering of the three-dimensional preoperative data is limited to at least one predefined distance zone. This allows to extract and use only information within the predefined distance zone. This allows to set the focus of the rendered image to the predefined distance zone. In particular, this is useful if only this predefined distance zone is relevant to a surgery, a treatment and/or an examination. Unwanted distraction by features outside the predefined distance zone may thus be averted. The predefined distance zone(s) may be set manually by the surgeon before or during the surgery or automatically by the medical visualization system, for example, by analyzing the three-dimensional preoperative data.

In one embodiment the rendering style of the three-dimensional preoperative data is set at least in part based on at least one predefined distance zone. This allows to define a rendering style and/or individual rules of the rendering style on the basis of predefined distance zones. For example, this allows to abruptly change a color and/or an opacity or transparency depending on the definition of distance zones. In particular, this allows specifically marking borders within the three-dimensional preoperative data. The predefined distance zone(s) may be set manually by the surgeon before or during the surgery or automatically by the medical visualization system, for example, by analyzing the three-dimensional preoperative data or by taking into account the properties of the medical visualization system, for example the depth of field or a multiple of it.

In one embodiment the rendering style is set at least in part based on a sign of the determined distance values. This allows to mark regions on both sides of interfaces between the at least one layer of the three-dimensional topography data and the three-dimensional preoperative data. For example, this embodiment may be used to indicate an interface between a part of a tumor that has already been removed (e.g. corresponding to negative distance values above a surface of the body) during surgery and a part of the tumor that has not been removed yet (e.g. corresponding to positive distance values below the surface of the body). This way, useful information regarding the size and location of the tumor and the progress of the surgery can be made visible. For example, the color of the features in the rendered image may be set to change abruptly with the sign value, e.g. yellow for the negative distances and red for the positive distances. The sign value may be directly derived from the determined distance values.

In one embodiment an interface between the at least one layer of the three-dimensional topography data and the three-dimensional preoperative data is rendered in a predetermined rendering style. This allows to mark the interface between the at least one layer of the three-dimensional topography data and the three-dimensional preoperative data. The interface may be rendered in a particular color or with a particular pattern (e.g. a grid). The coordinates corresponding with the interface may be determined by determining a set of points which corresponds to the intersection between the at least one layer of the three-dimensional topography data and the three-dimensional preoperative data, i.e. the coordinates of points which are included in both data sets.

In one embodiment, an outline of the three-dimensional preoperative data is projected onto the at least one layer of the three-dimensional topography data, and wherein the projected outline is included into the rendered image. This allows to provide additional information, in particular, for parts of the patient which correspond with the three-dimensional preoperative data and which are occluded by the at least one layer of the three-dimensional topography data, i.e. a surface between the parts and the viewer. In particular, the outline has the same extension as the feature from which the projection emanates, i.e. the projection does not change the scaling and is, in particular, a parallel projection.

In one embodiment, the rendered image comprises projection lines representing the projection of the outline onto the at least one layer of the three-dimensional topography data. This allows further indicating an occluded feature within the patient's body, in particular with respect to a distance below the occluding surface. In particular, the projection lines are oriented perpendicular to the outline.

Further features for embodiments of the medical visualization system may be derived from the description of embodiments of the method. The advantages of the medical visualization system are the same in each case as in the embodiments of the method.

The invention is explained in detail below on the basis of preferred exemplary embodiments with reference to the drawings. In the drawings:
- Fig. 1: shows a schematic to illustrate embodiments of the medical visualization system;
- Fig. 2: shows a schematic to illustrate an embodiment of the method and the medical visualization system;
- Fig. 3: shows a schematic to illustrate an embodiment of the method and the medical visualization system;
- Fig. 4: shows a schematic to illustrate an embodiment of the method and the medical visualization system;
- Fig. 5: shows a schematic to illustrate an embodiment of the method and the medical visualization system;
- Fig. 6: shows a schematic to illustrate an embodiment of the method and the medical visualization system;
- Fig. 7: shows a schematic to illustrate an embodiment of the method and the medical visualization system;
- Fig. 8: shows a schematic to illustrate an embodiment of the method and the medical visualization system;
- Fig. 9: shows a schematic to illustrate an embodiment of the method and the medical visualization system;
- Fig. 10: shows a schematic to illustrate an embodiment of the method and the medical visualization system;
- Fig. 11: shows a schematic to illustrate an embodiment of the method and the medical visualization system;
- Fig. 12: shows a schematic diagram to illustrate an embodiment of the method.

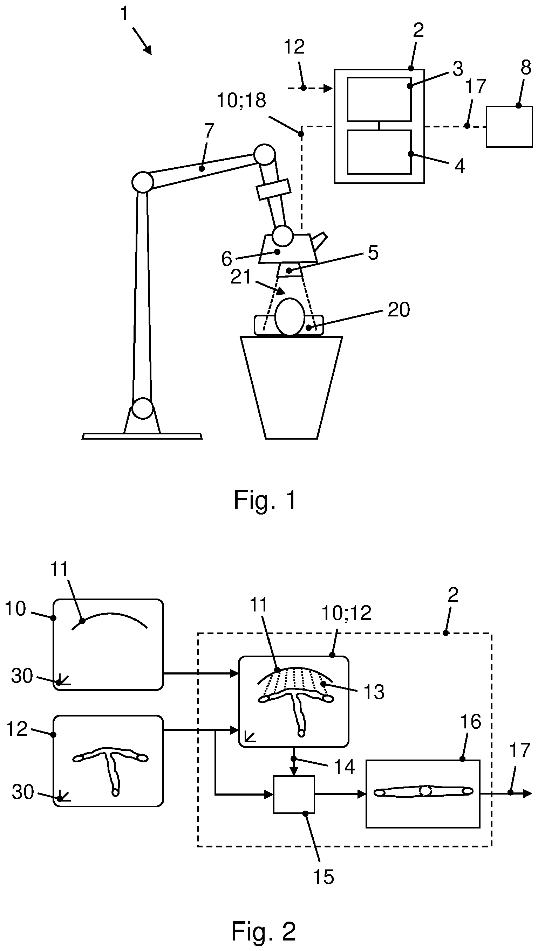

Fig. 1 shows a schematic of an embodiment of the medical visualization system 1. The medical visualization system 1 is, in the example shown, a surgical microscope. The medical visualization system 1 comprises a processing device 2. The processing device 2 comprises at least one processing unit 3, for example at least one microprocessor, and at least one memory 4. The processing device 2 is configured to execute at least part of the method described in this disclosure, and may therefor execute program code that is stored in the at least one memory 4 using the at least one processing unit 3. In the following, the method is described in more detail with the help of the medical visualization system 1.

The medical visualization system 1 may further comprise an image acquisition device 5 (e.g. a camera), in particular a stereoscopic image acquisition device (e.g. a stereoscopic camera) with a left channel and a right channel. The image acquisition device 5 may be configured to capture real images (e.g. white-light images) of a patient region of interest 21 of a patient 20.

The medical visualization system 1 may comprise a head 6, which is attached to a robotic stand 7 and may be moved manually or automatically by the robotic stand 7. The head 6 may carry the image acquisition device 5 (including the optics) of the medical visualization system 1.

The processing device 2 is configured to execute at least part of the method, the individual steps of which will be described in the following. The corresponding workflow is shown schematically in fig. 2. The processing device 2 is configured to receive at least one layer 11 of three-dimensional topography data 10 of the patient region of interest 21. In the following, only one layer 11 is mentioned, but the workflow would be the same for further layers. The at least one layer 11 corresponds, in particular to a layer of biological tissue or an anatomical surface of the patient 20, for example the skull, the dura, the brain and/or a tumor etc. Fig. 2 shows a cross-section of the three-dimensional topography data 10.

The three-dimensional topography data 10 may be captured and/or determined by using at least one sensor and/or a stereoscopic image acquisition device (e.g. a stereoscopic camera). For example, stereoscopic images may be analyzed in order to extract depth information of the at least one layer 11. Further, at least one sensor may be alternatively or additionally used to capture and/or determine the three-dimensional topography data 10 of the patient region of interest 21.

The processing device 2 receives three-dimensional preoperative data 12 of the patient region of interest 21. The three-dimensional preoperative data 12 may be CT or MRT data which was captured of the patient region of interest 21 before. Capturing the three-dimensional preoperative data 12 may also be part of the method.

It should be noted, that the three-dimensional topography data 10 and the three-dimensional preoperative data 12 are provided with respect to the same coordinate system 30. If they are not in the same coordinate system 30, then they are transformed into the same coordinate system 30. For this purpose, a transformation is determined, for example by using (patient) registration techniques commonly known in the art. An external or internal navigation system and/or surface matching techniques to match the three-dimensional preoperative data to a particular surface, e.g. within the patient region of interest 21, may be used to obtain the parameters of the transformation.

The processing device 2 is configured to determine distance values 14 of a distance 13 (only one distance 13 is marked for illustrative purposes in fig. 2) between the three-dimensional preoperative data 12 and the at least one layer 11 of the three-dimensional topography data 12. After the determination, a set of distance values 14 for each three-dimensional position or coordinate of the three-dimensional preoperative data 12 can be provided. The distance 13 or the distance values 14, respectively, are determined by using a predetermined metric. In particular, this may be a Euclidian metric with a specific rule for the direction in which the distance is determined in each case.

The processing device 2 is configured to generate a rendered image 16 by rendering the three-dimensional preoperative data 12 with a rendering style based at least in part on the determined distance values 14. In other words, the rendering style of each three-dimensional coordinate of the three-dimensional preoperative data 12 is set based on its corresponding distance value 14, i.e. its distance 13 to the at least one layer 11 of the three-dimensional topography data 10. A rendering module 15 may execute the rendering, determining the individual rendering style for each three-dimensional coordinate of the three-dimensional preoperative data 12 based on the corresponding distance value 14.

The rendered image 16 is, in particular, rendered from a predetermined perspective. This predetermined perspective corresponds, in particular, to a perspective of the image acquisition device 5 (e.g. a camera) of the medical visualization system 1 or a viewer.

The processing device 2 generates and provides an image signal 17, which represents the rendered image 16. In other words, the image signal 17 encodes the content of the rendered image 16. The image signal 17 may be displayed on a display 8 (fig. 1) of the medical visualization system 1. The display 8 may also be a projector with a glass plate located in an optical path of the medical visualization system 1.

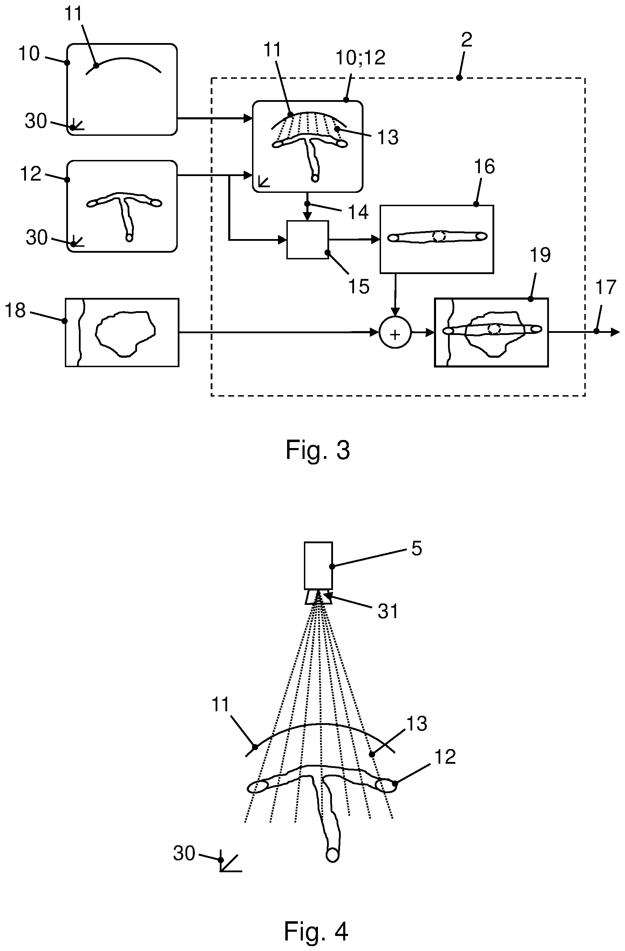

Fig. 3 shows a schematic to illustrate an embodiment of the method and the medical visualization device. In this embodiment, the processing device 2 may further be configured to receive a real image 18 (e.g. a white-light and/or RGB image) of the patient region of interest 21 and to merge the rendered image 17 with the real image 16, wherein the generated and provided image signal 17 represents the merged image 19. The real image 18 may be captured by the image acquisition device 5 (e.g. the camera) of the medical visualization system 1. The perspective from which the real image 18 is or was captured is the same perspective the rendered image 16 is rendered for. In particular, a pose (position and orientation) of the image acquisition device 5 (e.g. the camera) (fig. 1) is known or may be determined, such that the perspective may be provided as information for the rendering of the rendered image 16.

The real image 18 and the rendered image 16 may be merged by alpha blending. Each of the pixels of the rendered image 16 may have assigned an alpha value, which, in particular is set based on the respective distance values 14. The alpha values may be determined during the rendering by the rendering module 15.

Fig. 4 shows a schematic to illustrate an embodiment of the method and the medical visualization device. In this embodiment, the distance values 14 may be determined with respect to a predetermined viewpoint 31 of the medical visualization system 1. The predetermined viewpoint 31 is defined within the coordinate system 30. The predetermined viewpoint 31 may correspond to the viewpoint of the image acquisition device 5 (e.g. the camera) of the medical visualization system 1, in particular, the image acquisition device 5 (e.g. the camera), which captures the real image 18.

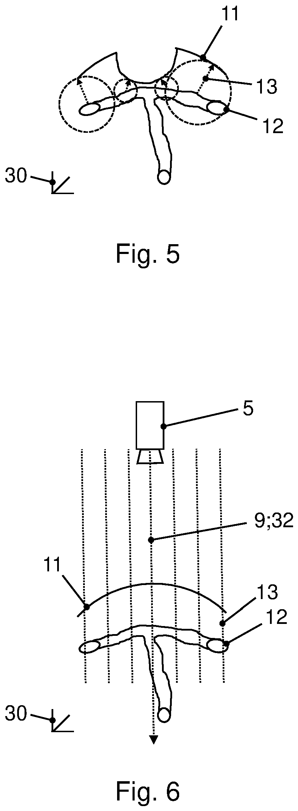

Fig. 5 shows a schematic to illustrate an embodiment of the method and the medical visualization device. In this embodiment, the distance values 14 (figures 2 and 3) may be determined as absolute distance 13 between the three-dimensional preoperative data 12 and the at least one layer 11 of the three-dimensional topography data 10. The absolute distance 13 refers to, in particular, to the distance of a point of the three-dimensional preoperative data 12 to the nearest point of the at least one layer 11 of the three-dimensional topography data. In particular, it refers to the Euclidean distance measured, for example in mm, between each point of the three-dimensional preoperative data 12 and its respective nearest point of the at least one layer 11 of the three-dimensional topography data. The nearest point may be found, for example, by encircling the respective point of the three-dimensional preoperative data 12 and increasing the radius of the circle until the circle first touches the at least one layer 11 of the three-dimensional topography data, as illustrated schematically in fig. 5. Then, the absolute distance 13 is the distance between the point and the touch point.

Fig. 6 shows a schematic to illustrate an embodiment of the method and the medical visualization device. In this embodiment, the distance values 14 may be determined along a direction parallel to a predetermined vector 32. The vector is defined in the coordinate system 30. In particular, the predetermined vector 32 runs parallel to an optical axis 9 of the medical visualization system 1. In particular the optical axis 9 is the optical axis of the image acquisition device 5 (e.g. the camera) of the medical visualization system 1.

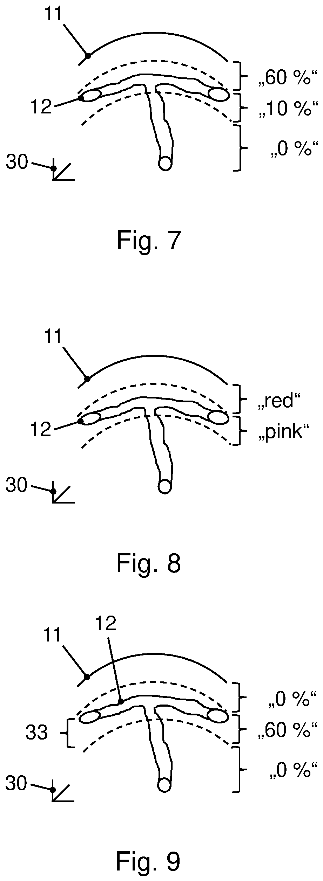

Fig. 7 shows a schematic to illustrate an embodiment of the method and the medical visualization device. In this embodiment, the rendering style may include an opacity or a transparency, wherein an opacity value or a transparency value is set at least in part based on the determined distance values 14. In the example shown, the rendering module 15 adapts the visibility of the rendered three-dimensional preoperative data 12. Up to a distance of a first preset distance value, for example 2 cm, the data 12 is rendered with a first preset opacity value, for example 60 %. Up to a distance of a second preset distance value, for example 4 cm, the data 12 is rendered with a second preset opacity value, for example 10 %. As the distance increases the data 12 is blended out and is therefore rendered with a third preset opacity value, for example 0 %, e.g. it is not rendered at all below the second preset distance value.

Fig. 8 shows a schematic to illustrate an embodiment of the method and the medical visualization device. In this embodiment, the rendering style may include a color, wherein a color value is set at least in part based on the determined distance values 14. In the example shown, the rendering module 15 adapts the color of the rendered three-dimensional preoperative data 12. Up to a distance of a first preset distance value, for example 2 cm, the data 12 is rendered with a first preset color value, for example color values for the color red. Up to a distance of a second preset distance value, for example 4 cm, the data 12 is rendered with a second preset color value, for example color values for the color pink. Below the second preset distance value the data 12 may be rendered in a third color or may not be rendered at all. It is also possible to blend between the color values based on the distance values when increasing or decreasing the distance.

The rendering style of the three-dimensional preoperative data 12 may be set at least in part based on at least one predefined distance zone 33 (fig. 9). This allows to define the rendering style for different distance zones 33.

Fig. 9 shows a schematic to illustrate an embodiment of the method and the medical visualization device. In this embodiment, the rendering of the three-dimensional preoperative data 12 may be limited to at least one predefined distance zone 33. The predefined distance zone 33 is, for example, defined to start at a distance of 2 cm and end at a distance of 4 cm. Within the predefined distance zone 33, the opacity value is set, for example, to a value of 60 %. Outside the predefined distance zone 33, the opacity value is set to 0 %, i.e. the data 12 is not rendered outside the predefined distance zone 33.

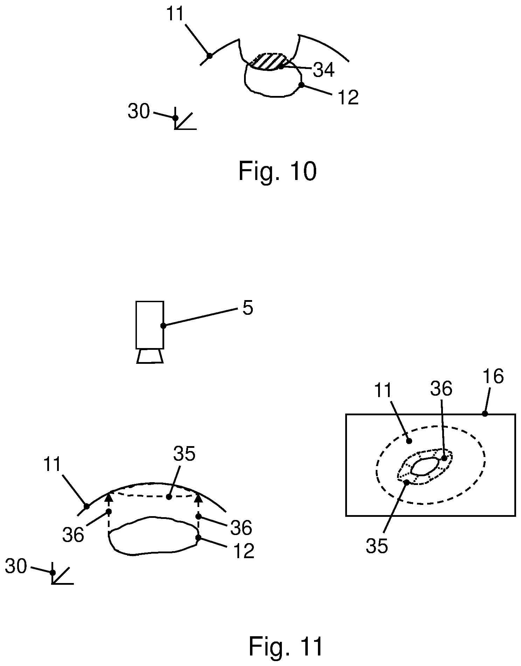

Fig. 10 shows a schematic to illustrate an embodiment of the method and the medical visualization device. In this embodiment, the rendering style may be set at least in part based on a sign of the determined distance values 14. The three-dimensional pre-operative data 12 represent, for example, a tumor. Part of the tumor has already been removed as part of an undergoing surgery, while the rest of the tumor is still within the body of the patient. The layer 11 of the three-dimensional topography data intersects with the tumor. The part above the layer 11 (marked with a striped pattern) has, for example, a negative sign, i.e. a negative distance to the layer 11. The part below the layer 11 (without the pattern) has, for example, a positive sign, i.e. a positive distance to the layer 11. The rendering style of the part above the layer 11 is then set to be different from the rendering style of the part below the layer 11. For example, an opacity or transparency may be set differently and/or a color value may be set differently.

An interface 34 (fig. 10) between the at least one layer 11 of the three-dimensional topography data and the three-dimensional preoperative data 12 may be rendered in a predetermined rendering style. For example, the interface may be rendered in a predetermined color and/or with a grid pattern.

Fig. 11 shows a schematic to illustrate an embodiment of the method and the medical visualization device. In this embodiment, an outline 35 of the three-dimensional preoperative data 12 may be projected onto the at least one layer 11 of the three-dimensional topography data, wherein the projected outline 35 is included into the rendered image 16. The projection is performed in the direction in which the respective distance is measured.

The rendered image 16 may comprise projection lines 36 representing the projection of the outline 35 onto the at least one layer 11 of the three-dimensional topography data.

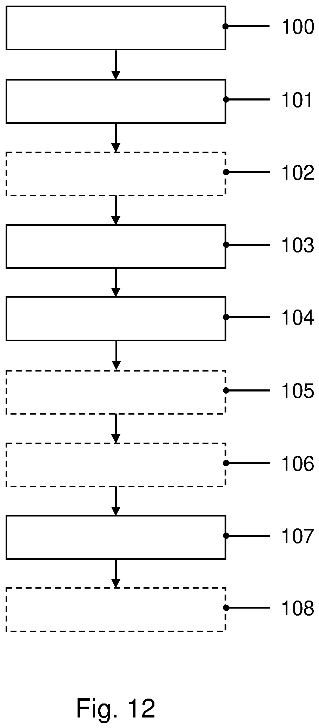

Fig. 12 shows a schematic diagram to illustrate embodiments of the method for providing an image signal for a medical visualization system. The medical visualization system may be the one described with respect to fig. 1.

In step 100, at least one layer of three-dimensional topography data of a patient region of interest is received. The three-dimensional topography data may be captured as part of step 100. For capturing the three-dimensional topography data a sensor of the medical visualization system may be used, for example a (stereoscopic) image acquisition device (e.g. a stereoscopic camera) or another depth sensitive senor. Alternatively, the three-dimensional topography data may have been captured beforehand and is simply accessed during step 100.

In step 101, three-dimensional preoperative data of the patient region of interest are received. The three-dimensional preoperative data may have been captured beforehand, for example by using CT or MRT or similar technologies.

In optional step 102, the three-dimensional topography data and the three-dimensional preoperative data are transformed into the same coordinate system, if necessary, i.e. they are not in the same coordinate system already. For this purpose a registration between the data may be performed.

In step 103, distance values of a distance between the three-dimensional preoperative data and the at least one layer of the three-dimensional topography data are determined. The distance values are, in particular, determined for every coordinate of the three-dimensional preoperative data. If more than one layer is defined, distance values for each of the layers are determined. The distance values are determined using a metric in the coordinate system, in particular a Euclidian metric.

In step 104, a rendered image is generated by rendering the three-dimensional preoperative data with a rendering style based at least in part on the determined distance values. The perspective of the rendered image corresponds, in particular, to the perspective of the real image captured by an image acquisition device (e.g. a camera) of the medical visualization system.

In optional step 105, a real image of the patient region of interest is received. The receiving may comprise capturing the real image of the patient region of interest with a (stereoscopic) image acquisition device (e.g. camera) of the medical visualization system.

In optional step 106, the rendered image is merged with the real image. The merging may comprise using alpha blending.

In step 107, an image signal which represents the rendered image or the merged image, respectively, is generated and provided.

In optional step 108, the image signal is displayed on a display of the medical visualization system.

Further embodiments of the method have already been described above with relation to the medical visualization system

### List of reference numerals

- 1: medical visualization system
- 2: processing device
- 3: processing unit
- 4: memory
- 5: (stereoscopic) image acquisition device (e.g. camera)
- 6: head
- 7: robotic stand
- 8: display
- 9: optical axis
- 10: three-dimensional topography data
- 11: layer
- 12: three-dimensional pre-operative data
- 13: distance
- 14: distance value
- 15: rendering module
- 16: rendered image
- 17: image signal
- 18: real image
- 19: merged image
- 20: patient
- 21: patient region of interest
- 30: coordinate system
- 31: predetermined viewpoint
- 32: predetermined vector
- 33: predefined distance zone
- 34: interface
- 35: outline
- 36: projection line
- 100-108: method steps

## Claims

1. Method for providing an image signal (17) for a medical visualization system (1), comprising:
receiving at least one layer (11) of three-dimensional topography data (10) of a patient region of interest (21),
receiving three-dimensional preoperative data (12) of the patient region of interest (21),
determining distance values (14) of a distance (13) between the three-dimensional preoperative data (12) and the at least one layer (11) of the three-dimensional topography data (10),
generating a rendered image (16) by rendering the three-dimensional preoperative data (12) with a rendering style based at least in part on the determined distance values (14), and
generating and providing an image signal (17) which represents the rendered image (16).

2. Method according to claim 1, wherein the method further comprises:
receiving a real image (18) of the patient region of interest (21),
merging the rendered image (16) with the real image (18),
wherein the generated and provided image signal (17) represents the merged image (18).

3. Method according to claim 1 or 2, wherein the distance values (14) are determined with respect to a predetermined viewpoint (31) of the medical visualization system (1).

4. Method according to claim 1 or 2, wherein the distance values (14) are determined as absolute distance between the three-dimensional preoperative data (12) and the at least one layer (11) of the three-dimensional topography data (10).

5. Method according to claim 1 or 2, wherein the distance values (14) are determined along a direction parallel to a predetermined vector (32).

6. Method according to claim 5, wherein the predetermined vector (32) runs parallel to an optical axis (9) of the medical visualization system (1).

7. Method according to one of the preceding claims, wherein the rendering style includes an opacity or a transparency, wherein an opacity value or a transparency value is set at least in part based on the determined distance values (14).

8. Method according to one of the preceding claims, wherein the rendering style includes a color, wherein a color value is set at least in part based on the determined distance values (14).

9. Method according to one of the preceding claims, wherein rendering of the three-dimensional preoperative data (12) is limited to at least one predefined distance zone (33).

10. Method according to one of the preceding claims, wherein the rendering style of the three-dimensional preoperative data (12) is set at least in part based on at least one predefined distance zone (33).

11. Method according to one of the preceding claims, wherein the rendering style is set at least in part based on a sign of the determined distance values (14).

12. Method according to one of the preceding claims, wherein an interface (34) between the at least one layer (11) of the three-dimensional topography data (10) and the three-dimensional preoperative data (12) is rendered in a predetermined rendering style.

13. Method according to one of the preceding claims, wherein an outline (35) of the three-dimensional preoperative data (12) is projected onto the at least one layer (11) of the three-dimensional topography data (10), and wherein the projected outline (35) is included into the rendered image (16).

14. Method according to claim 13, wherein the rendered image (16) comprises projection lines (36) representing the projection of the outline (35) onto the at least one layer (11) of the three-dimensional topography data (10).

15. Medical visualization system (1), comprising:
a processing device (2), configured to:
receive at least one layer (11) of three-dimensional topography data (10) of a patient region of interest (21),
receive three-dimensional preoperative data (12) of the patient region of interest (21),
determine distance values (14) of a distance (13) between the three-dimensional preoperative data (12) and the at least one layer (11) of the three-dimensional topography data (10),
generate a rendered image (16) by rendering the three-dimensional preoperative data (12) with a rendering style based at least in part on the determined distance values (14), and
generate and provide an image signal (17) which represents the rendered image (16).
